# EUROPEAN PATENT APPLICATION

(11) **EP 1 792 602 A1**
(43) Date of publication of application: **06.06.2007**
(21) Application number: 05775036.6
(22) Date of filing: 23.08.2005
(51) Int. Cl.: A61K 8/31, A61K 8/89, A61Q 19/00, A61Q 5/10

(54) **PRETREATING AGENT IN HAIR DYEING**

(30) Priority: 23.08.2004 JP 2004242331
(71) Applicant: Kikuboshi Corporation, Tokyo 111-0053 (JP); Sansho Cosme Inc., Osaka-shi, Osaka 558-0022 (JP)
(72) Inventor: HATANAKA, Katsuhito, iyoshi-ku, Osaka-shi, Osaka 5580022; (JP)
(74) Representative: Paget, Hugh Charles Edward
(86) International application number: PCT/JP2005/015286
(87) International publication number: WO 2006/022267

(57) **Abstract**

To provide a pretreatment agent for use in hair dyeing which is not thick and easy to be applied to scalp and little inhibits hair dyeing. A pretreatment agent for use in hair dyeing comprises polysiloxane, petrolatum and liquid paraffin as essential ingredients.

## Description

### Technical field

The present invention relates to pretreatment agents for use in hair dyeing.

### Background art

The wording "pretreatment in hair dyeing" refers to a treatment that is conducted on face, scalp, head hair or the like as a pretreatment in hair dyeing. This includes, for example, a protecting treatment executed for protecting skin from influence by hair dye, a hair dyeing pretreatment on head hair, and the like.

Since a hair dye typically uses alkaline or acidic liquid, it will inevitably give adverse affect on skin. In order to prevent this, it is desirable to apply a hair dye only on head hair but not to skin, which is however, unfeasible measure. When a hair dye adheres to skin, the skin is not only damaged by the aforementioned acid or alkaline but also colored.

In order to solve these problems, conventionally, a protecting agent is applied on skin prior to hair dyeing. As such a protecting agent, solid (cream) petrolatum or paraffin have been used.

Japanese patent application laid-open JP-A 2004-99515 discloses use of mixture of petrolatum and liquid paraffin as a protecting agent.

The protecting agent disclosed in JP-A 2004-99515 is designed for application to hairline of head hair. However, it is recently demanded of protection of scalp of the entire head as well as hairline. Although there arises the idea of applying the protecting agent disclosed in JP-A 2004-99515 on the scalp, the thick texture thereof makes application on scalp difficult. Of course, reduction of solids will reduce the protecting ability. Further, application of protecting agent on hair will inevitably inhibit hair dyeing.

### Disclosure of the invention

Therefore, it is an object of the present invention to provide a pretreatment agent for use in hair dyeing which will not give thick texture, and is easy to be applied to scalp and will little inhibit hair dyeing.

In consideration of the above circumstance, as a result of diligent effort, inventors of the present invention accomplished a pretreatment agent for use in hair dyeing of the present invention which is characterized by comprising polysiloxane, petrolatum and liquid paraffin as essential ingredients.

According to the present invention, there is provided a pretreatment agent for use in hair dyeing, which comprises polysiloxane, petrolatum and liquid paraffin as essential ingredients.
In the present invention, those additionally having a nonionic surfactant is preferred, and those additionally having a thermal stabilizer is further preferred.

The pretreatment agent for use in hair dyeing according to the present invention have the following advantages.
(1) Potently protecting skin from hair dye because of inclusion of petrolatum and polysiloxane.
(2) Too large quantity of petrolatum will make application to the entire head difficult, however, according to the present invention, inclusion of polysiloxane allows reduction in amount of petrolatum and facilitates application.
(3) By adjusting the quantities of petrolatum, polysiloxane and liquid paraffin, the dyeing power (ease of dyeing) may be adjusted.

### Best mode for carrying out the invention

In the following, the present invention will be explained more specifically based on the non limitative exemplary embodiments.

The pretreatment agent for use in hair dyeing according to the present invention comprises polysiloxane, petrolatum and liquid paraffin as essential ingredients.
Here, the term "polysiloxane" refers to those forming polymer with siloxane bond of SiO. Here, methyl polysiloxane is preferably used, however, modified siloxane may be used. Those having viscosity of 5 to 5000 CS, in particular, 100 to 500 CS are preferably used.

Polysiloxane is mixed so as to protect skin in a relatively small amount by the help of its water repellency. To be more specific, it is often the case that hair is first treated with an alkaline liquid in usual hair dyeing. This alkaline (aqueous) liquid is blocked by the water repellency. Of course, polysiloxane by itself is difficult to form cream and has difficulty in application and retention.

As the petrolatum, no special ones are required, but those commonly used in cosmetics may be used. Concretely, white petrolatum and the like may be used. In chemical aspects, solids having about 15 to 30 carbons may be used. The melting point is typically about 50 to 60°C.

As the liquid paraffin, no special ones are required. In chemical aspects, liquid hydrocarbons having about 15 to 30 carbons may be used.

A preferred mixing ratio of these three essential ingredients is: 50 to 300 parts by weight of liquid paraffin, relative to 100 parts by weight of petrolatum, and 0.1 to 5 parts by weight of polysiloxane, relative to 100 parts of mixture of liquid paraffin and petrolatum.
A preferred mixing ratio of petrolatum and liquid paraffin will lead cream form (paste form), and as such, the level previously described is preferred although it differs depending on the petrolatum in use and melting point of the liquid paraffin. Also, the mixing amount of polysiloxane differs depending on its molecular weight and viscosity, however, the above described mixing amount is preferred in the case of dimethyl polysiloxane which is commonly used for cosmetics.

A surfactant may be mixed with the above essential ingredients. This provides refreshing wash finish in washing-off after hair dyeing. As the surfactant, nonionic ones having HLB value of about 5 to 20 are preferred. Of course, commercially available ones may be used. A preferred mixing amount is about 2 to 15% by weight of the entirety.

Further, since the viscosity and shape retention vary with the ambient temperature, a thermal stabilizer may be mixed in order to reduce such variations. As the thermal stabilizer, hydrocarbons, esters and the like having a melting point of 50°C or higher are preferred. Behenyl alcohol, microcrystalline wax, glycerin and the like fatty acid esters can be exemplified.
Basically, the main object is to prevent liquefaction in summer or upon exposure to high temperature. A preferred mixing amount is about 5 to 25% by weight of the entirety.

Further, an antioxidant such as tocopherol may be mixed.

Further, other ingredients may be mixed without departing from the spirit and scope of the present invention. For example, colorant, UV absorber, anti-inflammatory agent, and other various bioactive ingredients may be mixed.
As an anti-inflammatory agent, arnica extract, chamomile extract, Scutellaria root extract, licorice extract, perilla extract, Mulberry bark extract, peach leaf extract, Saxifraga stolonifera extract, anthemis nobilis flower extract and the like can be exemplified.

### Examples

In the following, the present invention will be explained more specifically by way of examples.

### (Example 1)

The following ingredients were heated to 70°C and mixed well, and cooled to 30°C to make a cream product. The viscosity at 30°C was 50000 to 150000 CS.
Petrolatum: 39.9% by weight
Liquid paraffin: 30.0% by weight
Polysiloxane: 0.1% by weight (methyl polysiloxane)
Nonionic activator: 10.0% by weight (POE lanoline alcohol: 10EO)
Thermal stabilizer: 20.0% by weight (paraffin wax)

### (Example 2)

Petrolatum: 37.0% by weight
Liquid paraffin: 30.0% by weight
Polysiloxane: 3.0% by weight (methylpolysiloxane)
Nonionic activator: 10.0% by weight (POE lanoline alcohol: 5EO)
Thermal stabilizer: 20.0% by weight (microcrystalline wax)
Viscosity: 50000 to 150000 CS

### (Example 3)

Petrolatum: 20.0% by weight
Liquid paraffin: 60.0% by weight
Polysiloxane: 3.0% by weight
Nonionic activator: 7.0% by weight (POE hardened castor oil: 5EO)
Thermal stabilizer: 10.0% by weight (microcrystalline wax)
Viscosity: 3500 to 5500 CS

### (Comparative example 1)

Petrolatum: 50.0% by weight
Liquid paraffin: 20.0% by weight
Nonionic activator: 10.0% by weight (POE lanoline alcohol: 5EO)
Thermal stabilizer: 20.0% by weight (microcrystalline wax)
Viscosity: 200000 to 400000 CS

First, each of the cream products of Examples 1 to 3 was applied on scalp of the entire head.
The applied amount was about 30 g. The cream was applied to hairline with the use of a bottle container equipped with a nozzle.
The cream of Comparative example 1 was applied in almost the same manner.

For Examples 1 to 3 and Comparative example 1, since quantity of each ingredient was adjusted to ensure protectivity against skin, influence on skin after hair dyeing was comparable among these examples, and sufficient function was exerted as a protecting agent. Creams of Examples 1 to 3 could be readily applied and applied thinly without occurrence of dripping. Contrarily, the cream of Comparative example 1 had high viscosity, and could not be readily applied.

Further, creams of Examples 1 to 3 did not inhibit hair dyeing, and difficultly in dyeing was not observed. However, cream of Comparative example 1 showed somewhat poor dyeability.

### Industrial applicability

The pretreatment agent of the present invention is desirably used in hair dyeing because it is not thick, and hence easy to be applied to scalp, and little inhibits the hair dying.

## Claims

1. A pretreatment agent for use in hair dyeing , which comprises polysiloxane, petrolatum and liquid paraffin as essential ingredients.

2. The pretreatment agent for use in hair dyeing according to claim 1, further including a nonionic surfactant.

3. The pretreatment agent for use in hair dyeing according to claim 1, further including a thermal stabilizer.
